# EUROPEAN PATENT SPECIFICATION

(11) **EP 2 512 410 B1**
(45) Date of publication and mention of the grant of the patent: **03.06.2015**
(21) Application number: 10801502.5
(22) Date of filing: 17.12.2010
(51) Int. Cl.: A61K 8/25, A61Q 11/00

(54) **HIGH CLEANING AND LOW ABRASION SILICA MATERIALS TO MODULATE ENTRAPMENT AND RELEASE OF HYDROPHOBIC ACTIVES IN DENTAL FORMULATIONS**
SILICAMATERIALIEN MIT HOHER REINIGUNGSKRAFT UND GERINGER ABRASION ZUR MODULIERUNG DES EINSCHLUSSES UND DER FREISETZUNG VON HYDROPHOBEN WIRKSTOFFEN IN DENTALEN FORMULIERUNGEN
MATÉRIAUX SILICEUX À CAPACITÉ ÉLEVÉE DE NETTOYAGE ET FAIBLE CAPACITÉ D'ABRASION POUR MODULATION DE PIÉGEAGE ET DE LIBÉRATION D'AGENTS ACTIFS HYDROPHOBES DANS DES PRÉPARATIONS DENTAIRES

(30) Priority: 17.12.2009 US 287300 P
(43) Date of publication of application: 24.10.2012
(73) Proprietor: Colgate-Palmolive Company, New York, NY 10022 (US)
(72) Inventor: PIMENTA, Paloma, Staten Island, NY 10306 (US); PILCH, Shira, Highland Park NJ 08904 (US); MASTERS, James, Ringoes NJ 08551 (US)
(74) Representative: Jenkins, Peter David
(86) International application number: PCT/US2010/060975
(87) International publication number: WO 2011/084676

(56) References cited:
- WO-A1-2009/140577
- US-A- 5 658 553
- US-A1- 2006 110 338
- US-A1- 2008 159 967

## Description

### CROSS REFERENCE TO RELATED APPLICATIONS

This application claims priority to U.S. Provisional Patent Application No. 61/287,300, filed on 17 December 2009.

### FIELD OF THE INVENTION

The present embodiments relate to dentifrice compositions, and more particularly, dentifrice compositions containing high cleaning and low abrasion silica materials to control the retention and delivery of flavor compounds. The present embodiments also relate to dentifrice compositions containing high cleaning and low abrasion silica materials to control the retention and delivery of effective amounts of actives and/or remineralizing agents.

This invention relates to unique dentifrice formulations containing abrasives in the form of *in situ* generated compositions of precipitated silicas and gel silicas. These dentifrices exhibit various beneficial characteristics based on the particular structure and surface chemistry of the high cleaning and low abrasion hybrid gel/silica abrasives they contain. Depending on the particular parameters of the hybrid gel/silica abrasives, this invention exhibits unexpected beneficial qualities such as enhanced flavor retention and superior modulation of actives.

### BACKGROUND

A tooth is comprised of an inner dentin layer and an outer hard enamel layer that is the protective layer of the tooth. The enamel layer of a tooth is naturally an opaque white or slightly off-white color. It is this enamel layer that can become stained or discolored. The enamel layer of a tooth is composed of hydroxyapatite mineral crystals that create a somewhat porous surface. It is believed that this porous nature of the enamel layer is what allows staining agents and discoloring substances to permeate the enamel and discolor the tooth

Many substances that a person confronts or comes in contact with on a daily basis can "stain" or reduce the "whiteness" of their teeth. In particular, the foods, tobacco products and fluids such as tea and coffee that one consumes tend to stain the teeth. These products or substances tend to accumulate on the enamel layer of the tooth and form a pellicle film over the teeth. These staining and discoloring substances can then permeate the enamel layer. This problem occurs gradually over many years, but imparts a noticeable discoloration of the enamel of one's teeth.

Abrasive substances have historically been used in dentifrice compositions to remove various deposits and substances that can cause such tooth discoloration. These deposits and substances sometimes come in the form of pellicle film, which tightly adheres to the tooth and causes a yellow or brown appearance. Silica dental abrasives of various types are preferred because of their unique benefits of exceptional dental cleaning and polishing performance without unduly abrading tooth enamel or dentin.

With traditional silica materials, a strong relationship exists between mechanical stain removal and abrasion, with incremental gains in cleaning performance resulting in significant increase in dentin abrasivity. The abrasive, when cleaning, should not harm the tooth. Thus, standard approaches to mechanically remove stain are reaching their limits.

Trends facilitated by industry leaders have focused on an attempt to convince the profession that dentifrice abrasive properties can facilitate tooth-wear, especially during prolonged exposure to acid from dietary sources. The current limitations of novel high cleaning silica technologies have resulted in a new generalized market need for the development of high cleaning materials that provide superior stain removing, while supporting a low level of abrasivity in whitening products.

Flavor is one of the more important attributes of any product that is applied or ingested orally, including but not limited to foods, drinks, medications and oral hygiene products, such as toothpaste. The golden years of flavor research in the United States were the 1960's and 1970's, mostly within the food industry [Reineccius, G. (1999) Sourcebook of Flavors, 2nd Edition. Aspen Publishers Inc. ] and since then numerous methodologies have been made available to quantify flavor compositions and flavor release characteristics. In all products the raw materials used contribute their own chemistry and may interact differently with flavor compounds, therefore flavor release profiling becomes a typical step in the development and/ or substitution of any raw material in a given product.

The ideal next generation high cleaning dentifrice should possess some uniqueness in terms of its flavor release profile, retention and intensity. The next generation dentifrice also should have sensory mouthfeel attributes and/or an additional therapeutic benefit.

U.S. Pat. No. 7,267,814 discloses dentifrice compositions icncluding in situ produced gel/ precipitate silica composites comprising 10 to 60% by volume of silica gel with a linseed oil absorption between 100 and 150 mL/100g.

U.S. Pat. No. 7,303,742 also discloses dentifrice compositions including in situ produced gel/ precipitate silica composites. The composites are comprised of 20 to 85% by volume of silica gel with a linseed oil absorption between 100 and 150 mL/100g. Also disclosed are various dentifrice compositions containing such abrasives that exhibit a PCR : RDA (Pellicle Cleaning Ratio: Radioactive Dentin Abrasion) ratio of from about 0.80 to about 3.5, a PCR value between about 50 and 80, and a RDA level of between about 20 and about 80.

US Patent Application 2006/0140878 (filed Jun 29, 2006) discloses dentifrice compositions comprised of amorphous precipitated silica particles, wherein the silica particles exhibit a linseed oil absorption between 50 and 90 mL/100g. In addition, this application discloses dentifrice compositions above exhibiting a RDA level between about 130 and 200 and a PCR of between about 100 and 140, and a PCR:RDA ratio between 0.65 and 1.1.

US Patent 7,306,788 discloses an in situ produced gel/precipitate silica composite, wherein the composite is comprised of from 5 to 50% by volume of silica gel with a PCR:RDA (Pellicle Cleaning Ratio: Radioactive Dentin Abrasion) ratio of from about 0.45 to about 0.7, a PCR value between about 90 and 160, and a RDA level of at most 240.

The description herein of certain advantages and disadvantages of known compositions, methods, and apparatus is not intended to limit the scope of the embodiments to their exclusion (or inclusion, as the case may be). Indeed, certain embodiments may include one or more known compounds, methods, or apparatus without suffering from the afore-mentioned disadvantages.

### SUMMARY

This invention embodies dentifrices utilizing hybrid silicas with properties that are intermediate between low-structure precipitated silicas (abrasive) and higher structure gel-like silicas (thickening), and that may be utilized to modulate the retention and release of hydrophobic compounds to either improve organoplectic acceptability or to provide novel therapeutic and/or sensory benefits.

Some embodiments make use of hybrid precipitated/ gel-like silicas that are prone to retaining hydrophobic aromatic compounds such as flavor components, which subsequently may be released during brushing, causing an unusual flavor-related sensory perception. Some embodiments also may contain an effective amount of a remineralizing agent built into the silica surface to be deposited on the tooth surface upon brushing.

Other embodiments may contain an effective amount of suitable cosmetic and/or therapeutic actives. Such actives include any material that is generally considered safe for use in the oral cavity and that provides changes to the overall appearance and/or health of the oral cavity, including, but not limited to, anti-calculus agents, fluoride ion sources, stannous ion sources, whitening agents, antimicrobial, anti-plaque agents, anti-inflammatory agents, nutrients, antioxidants, anti-viral agents, analgesic and anesthetic agents, H-2 antagonists, and mixtures thereof. When present, the level of cosmetic and/ or therapeutic active in the dentifrice is, in one embodiment from about 0.001% to about 90%, in another embodiment from about 0.01% to about 50%, and in another embodiment from about 0.1% to about 30%, by weight of the dentifrice.

### DETAILED DESCRIPTION

The following definitions and non-limiting guidelines should be considered in reviewing the description of the invention set forth herein. The headings (such as "Background" and "Summary") and sub-headings used herein are intended only for general organization of topics within the disclosure of the invention, and are not intended to limit the disclosure of the invention or any aspect thereof. In particular, subject matter disclosed in the "Background" may include aspects of technology within the scope of the invention, and may not constitute a recitation of prior art. Subject matter disclosed in the "Summary" is not an exhaustive or complete disclosure of the entire scope of the invention or any embodiments thereof. Classification or discussion of a material within a section of this specification as having a particular utility (e.g., as being an "active" or a "carrier" ingredient) is made for convenience, and no inference should be drawn that the material must necessarily or solely function in accordance with its classification herein when it is used in any given composition.

The citation of documents does not constitute an admission that those documents are prior art or have any relevance to the patentability of the invention disclosed herein. Any discussion of the content of documents cited in the Introduction is intended merely to provide a general summary of assertions made by the authors of the documents, and does not constitute an admission as to the accuracy of the content of such documents.

The description and specific examples, while indicating embodiments of the invention, are intended for purposes of illustration only and are not intended to limit the scope of the invention. Moreover, recitation of multiple embodiments having stated features is not intended to exclude other embodiments having additional features, or other embodiments incorporating different combinations of the stated features. Specific examples are provided for illustrative purposes of how to make and use the compositions and methods of this invention and, unless explicitly stated otherwise, are not intended to be a representation that given embodiments of this invention have, or have not, been made or tested.

As used herein, the words "preferred" and "preferably" refer to embodiments of the invention that afford certain benefits, under certain circumstances. However, other embodiments also may be preferred, under the same or other circumstances. Furthermore, the recitation of one or more preferred embodiments does not imply that other embodiments are not useful, and is not intended to exclude other embodiments from the scope of the invention.

As used herein, "comprising" encompasses "consisting of" and "consisting essentially of." As used herein, the word "include," and its variants, is intended to be non-limiting, such that recitation of items in a list is not to the exclusion of other like items that may also be useful in the materials, compositions, devices, and methods of this invention.

As used herein, the term "about" when applied to the value for a parameter of a composition or method of this invention, indicates that the calculation or the measurement of the value allows some slight imprecision without having a substantial effect on the chemical or physical attributes of the composition or method. If, for some reason, the imprecision provided by "about" is not otherwise understood in the art with this ordinary meaning, then "about" as used herein indicates a possible variation of up to 5% in the value.

All percentages used herein are by weight of the total dentifrice composition, unless otherwise specified. The ratios used herein are weight ratios of the respective components, unless otherwise specified. All measurements are made at 25°C, unless otherwise specified.

As used throughout, ranges are used as a shorthand for describing each and every value that is within the range. Any value within the range can be selected as the terminus of the range.

Herein, "effective amount" means an amount of a compound or composition sufficient to significantly induce a positive benefit, preferably an oral health benefit, but low enough to avoid serious side effects, i.e., to provide a reasonable benefit to risk ratio, within the sound judgment of a skilled artisan.

A dentifrice composition is a product, which in the ordinary course of administration, is not intentionally swallowed for purposes of systemic administration of particular therapeutic agents, but is rather retained in the oral cavity for a time sufficient to contact substantially all of the tooth surfaces and/or oral tissues for purposes of oral activity. A dentifrice composition of the present invention may be in the form of a toothpaste or dentifrice. The term "dentifrice," as used herein, means paste or gel formulations unless otherwise specified. The dentifrice composition may be in any desired form, such as deep striped, surface striped, multi-layered, having the gel surrounding the paste, or any combination thereof.

While the specification concludes with claims particularly pointing out and distinctly claiming the invention, it is believed that the present invention will be better understood from the following description of preferred embodiments.

This invention embodies the use of high cleaning and low abrasion silica materials to control the retention and delivery of flavor compounds that result in unique sensory perception of flavors during brushing. The in situ formation of the precipitate/gel hybrid silica materials, preferably followed by a specifically controlled milling process, preferably provides the silica with a particle size (e.g., median particle size of about 5-30 microns, preferably about 9-14 microns), and a ratio of accessible to total pore volume of silica (CTAB (cetyl trimethyl ammonium bromide) surface area of 60-110 m2/g, Oil Absorption of 80-130 cc/ 100 g, CTAB/OA ratio of 0.7 to 1) that enables them to entrap an unusually high level of hydrophobic compounds. The CTAB surface area is from 85 to 110 m2/g. This ability of the silica results in modulating the release of flavors thereby providing significant impact on consumer perception during brushing. At the same time, the silicas provide a high clean and low abrasion performance relative to a conventional high clean silica.

The entrapment of flavor can further be enhanced by the unique colloidal network (5 times the G' value relative to a conventional high cleaning silica (HCS) within the linear viscoelastic region at 20% loading) formed by the hybrid silicas.

Synthetic precipitated silica powders generally are produced by the destabilization and precipitation of amorphous silica from soluble alkaline silicate by the addition of mineral acid or acid gases. Under such conditions, primary particles are formed and subsequently associate with each other to form aggregates of various sizes [Davis, W. B., and Winter, P. J., (1976) "Measurement In vitro of Enamel Abrasion by Dentifrice", J. Dent. Res., 55: 970]. The morphology and pore structure of the precipitated particles and aggregates are highly dependent on the specific reaction conditions. The resulting precipitate is separated from the aqueous fraction of the reaction mixture by filtering, washing, and drying procedures, and then the dried product is mechanically milled in order to provide a suitable particle size and size distribution.

All steps are believed to contribute to the physical and chemical properties of the final product, so that modifications to the silica pore structure and morphology can be achieved through variation in any aspect of the production process, e.g. reagents used, rate of reaction, filtering and drying, and milling. The downsides of some of the modifications are, for example an increase in abrasion or, on the other hand, a change in structural properties that causes a rheological imbalance, requiring reformulation. A person having ordinary skill in the art will be capable of modifying the processing steps described herein to vary the physical and chemical properties of the silica, using the guidelines provided herein.

The silica materials that are commonly used in dentifrice formulations are not so specific in terms of pore size distribution and surface chemistry, as precise control over these attributes tends to increase the material costs significantly. As a result, commercial oral care grade silicas contain a wide pore size distribution which includes micropores (<2nm), mesopores (2-50nm) and macropores (> 50nm). The mesopores and macropores are the 'accessible surface area' of the silica, and retention and subsequent delivery of actives would be controlled by the morphology and surface properties at these length scales.

One of the preferred silicas utilized in the present invention is unlike conventional precipitated silicas described above in that it is a combination of an in situ formation of gel and precipitated silica. Such silica materials are described in, for example, U.S. Patent Application Publication Nos. 2006/0110338, 2006/0110307, 2006/0110339, 2006/0110336, and 2006/0140878. The in situ formed gel and precipitated silica particles can be made by any of the methods disclosed in the afore-mentioned documents. In this process, a silica gel is formed first, through addition of acid to silicate without washing or purification. In a subsequent step within the same process, a precipitated silica material is created through additional introduction of silicate and acid material. The methodology of creating this hybrid gelled and precipitated silica material within the same reaction vessel before washing, drying and milling provides the flexibility to create a range of silica products of different cleaning and abrasive capabilities by controlling the gel to precipitate ratio. The result is a material that has distinct physical properties, e.g. pore structure and morphology.

The invention is a dentifrice containing the above described silica. Such an embodiment may contain, for example, 10-30% by weight silica, prepared in a 0.2% aqueous Xanthan gum solution in order to maintain homogeneity for at least several days. Then, a concentration of 0.5% by weight of a flavor is added followed by addition of 0.5% by weight sodium lauryl sulfate (SLS), used to keep the flavor emulsified.

In the preferred embodiment, the flavor compound would exhibit a logarithmic partition (log P) value of 2.0 or greater. The Partition (P) coefficient is the ratio of concentrations of a compound in the two phases of a mixture of two immiscible solvents at equilibrium. Hence this coefficient is a measure of differential solubility of the compound between these two solvents. In the case of flavorants, the higher the P (and logarithmic P), the greater the hydrophobic nature of the flavor compound.

In a preferred embodiment of the present invention, the flavorant is a combination of one or more of the following flavor compounds that exhibiting a log P of 2.0 or greater: Carvone Laevo, Cinnamic Aldehyde, Eugenol, Menthol, Methyl Salicylate, Eucalyptol, Menthone, Isomenthone, Anethole, Menthyl Acetate, Limonene. See Table 2 for particular specifications of these, and other,. flavorants.

The dentifrice compositions of the present invention could preferably incorporate a remineralizing agent "built into" the silica Potential applications in the delivery of therapeutic actives also exist. An example is the delivery of actives for the prevention and mitigation of tooth ware. The hybrid is an ideal silica material to employ in such an application, since it provides high cleaning with low abrasion and capability to house actives within its large pore volume. Remineralizing agents can be 'built into' the silica surface to be deposited on the tooth surface upon brushing. A similar approach has been employed in re-mineralization of bone tissue for bone replacement and repair.

The present invention also may contain an effective amount of one or more humectants. A humectant can help to keep the dentifrice composition from hardening upon exposure to air, and it may provide a moist feel in the mouth. Suitable humectants for use in the present invention include water, edible polyhydric alcohols such as glycerin, sorbitol, xylitol, butylene glycol, polyethylene glycol, propylene glycol, and combinations thereof. Sorbitol, glycerin, water, and combinations thereof are preferred humectants. The humectant may be present in an amount of from about 0.1 % to about 99%, from about 0.5% to about 95%, and from about 1% to about 90%.

The binder system may further comprise additional inorganic thickening agents such as colloidal magnesium aluminum silicate or finely divided silica to further improve texture. Additional inorganic thickening agents if present can be used in an amount from about 0.1 % to about 15%, more preferably from about 0.1% to about 5%, by weight of the dentifrice composition.

The present invention also may include a peroxide source in the composition. The peroxide source may be selected from the group consisting of hydrogen peroxide, calcium peroxide, urea peroxide, and mixtures thereof. The preferred peroxide source is calcium peroxide. The following amounts represent the amount of peroxide raw material, although the peroxide source may contain ingredients other than the peroxide raw material. The present composition may contain from about 0.01% to about 10%, preferably from about 0.1% to about 5%, more preferably from about 0.2% to about 3%, and most preferably from about 0.3% to about 0.8% of a peroxide source, by weight of the dentifrice composition.

The compositions also may include an alkali metal bicarbonate salt. Alkali metal bicarbonate salts are soluble in water and unless stabilized, tend to release carbon dioxide in an aqueous system. Sodium bicarbonate, also known as baking soda, is the preferred alkali metal bicarbonate salt. The alkali metal bicarbonate salt also functions as a buffering agent. The present composition may contain from about 0.5% to about 50%, preferably from about 0.5% to about 30%, more preferably from about 2% to about 20%, and most preferably from about 5% to about 18% of an alkali metal bicarbonate salt, by weight of the dentifrice composition.

The compositions also may comprise surfactants, also commonly referred to as sudsing agents. Suitable surfactants are those that are reasonably stable and foam throughout a wide pH range. The surfactant may be anionic, nonionic, amphoteric, zwitterionic, cationic, or mixtures thereof. Anionic surfactants useful herein include the water-soluble salts of alkyl sulfates having from 8 to 20 carbon atoms in the alkyl radical (e.g., sodium alkyl sulfate) and the water-soluble salts of sulfonated monoglycerides of fatty acids having from 8 to 20 carbon atoms. Sodium lauryl sulfate and sodium coconut monoglyceride sulfonates are examples of anionic surfactants of this type. Other suitable anionic surfactants are sarcosinates, such as sodium lauroyl sarcosinate, taurates, sodium lauryl sulfoacetate, sodium lauroyl isethionate, sodium laureth carboxylate, and sodium dodecyl benzenesulfonate. Mixtures of anionic surfactants can also be employed. Many suitable anionic surfactants are disclosed by Agricola et al., U.S. Pat. No. 3,959,458, issued May 25,1976.

Nonionic surfactants that can be used in the compositions can broadly be defined as compounds produced by the condensation of alkylene oxide groups (hydrophilic in nature) with an organic hydrophobic compound which may be aliphatic or alkyl-aromatic in nature. Examples of suitable nonionic surfactants include poloxamers (sold under trade name PLURONIC^{®}), polyoxyethylene, polyoxyethylene sorbitan esters (sold under trade name TWEENS^{®}), Polyoxyl 40 hydrogenated castor oil, fatty alcohol ethoxylates, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides, and mixtures of such materials. The amphoteric surfactants useful in the present invention can be broadly described as derivatives of aliphatic secondary and tertiary amines in which the aliphatic radical can be a straight chain or branched and wherein one of the aliphatic substituents contains from about 8 to about 18 carbon atoms and one contains an anionic water-solubilizing group, e.g., carboxylate, sulfonate, sulfate, phosphate, or phosphonate. Other suitable amphoteric surfactants are betaines, specifically cocamidopropyl betaine. Mixtures of amphoteric surfactants can also be employed. Many of these suitable nonionic and amphoteric surfactants are disclosed by Gieske et al. in U.S. Pat. No. 4,051,234. The present composition typically comprises one or more surfactants each at a level of from about 0.25% to about 12%, preferably from about 0.5% to about 8%, and most preferably from about 1% to about 6%, by weight of the composition.

Titanium dioxide may also be added to the present composition. Titanium dioxide is a white powder which adds opacity to the compositions. Titanium dioxide generally comprises from about 0.25% to about 5%, by weight of the composition.

Coloring agents may also be added to the present composition. The coloring agent may be in the form of an aqueous solution, preferably 1 % coloring agent in a solution of water. Color solutions generally comprise from about 0.01% to about 5%, by weight of the composition.

Sweetening agents can be added to the compositions. These include saccharin, dextrose, sucrose, lactose, xylitol, maltose, levulose, aspartame, sodium cyclamate, D-tryptophan, dihydrochalcones, acesulfame, and mixtures thereof. Various coloring agents may also be incorporated in the present invention. Sweetening agents and coloring agents are generally used in toothpastes at levels of from about 0.005% to about 5%, by weight of the composition.

The present invention may also include other agents, such as antimicrobial agents. Included among such agents are water insoluble non-cationic antimicrobial agents such as halogenated diphenyl ethers, phenolic compounds including phenol and its homologs, mono and poly-alkyl and aromatic halophenols, resorcinol and its derivatives, bisphenolic compounds and halogenated salicylanilides, benzoic esters, and halogenated carbanilides, polyphenols, and herbals. The water soluble antimicrobials include quaternary ammonium salts and bis-biquanide salts, among others. Triclosan monophosphate is a preferred additional water soluble antimicrobial agent. The quaternary ammonium agents include those in which one or two of the substitutes on the quaternary nitrogen has a carbon chain length (typically alkyl group) from about 8 to about 20, typically from about 10 to about 18 carbon atoms while the remaining substitutes (typically alkyl or benzyl group) have a lower number of carbon atoms, such as from about 1 to about 7 carbon atoms, typically methyl or ethyl groups. Dodecyl trimethyl ammonium bromide, tetradecylpyridinium chloride, domiphen bromide, N-tetradecyl-4-ethyl pyridinium chloride, dodecyl dimethyl (2-phenoxyethyl) ammonium bromide, benzyl dimethylstearyl ammonium chloride, cetyl pyridinium chloride, quaternized 5-amino-1,3-bis(2-ethyl-hexyl)-5-methyl hexa hydropyrimidine, benzalkonium chloride, benzethonium chloride and methyl benzethonium chloride are examplary of typical quaternary ammonium antibacterial agents. Other compounds are bis[4-(R-amino)-1-pyridinium] alkanes as disclosed in U.S. Pat. No. 4,206,215, issued Jun. 3,1980, to Bailey.

Other antimicrobials such as copper bisglycinate, copper glycinate, zinc citrate, and zinc lactate may also be included. Also useful are enzymes, including endoglycosidase, papain, dextranase, mutanase, and mixtures thereof. Such agents are disclosed in U.S. Pat. No. 2,946,725, Jul. 26,1960, to Norris et al. and in U.S. Pat. No. 4,051,234, Sep. 27,1977 to Gieske et al. Specific antimicrobial agents include chlorhexidine, triclosan, triclosan monophosphate, and flavor oils such as thymol. Triclosan is a preferred antimicrobial agent for inclusion in the present compositions. Triclosan and other agents of this type are disclosed in Parran, Jr. et al., U.S. Pat. No. 5,015,466, issued May 14,1991, and U.S. Pat. No. 4,894,220, Jan. 16, 1990 to Nabi et al. The water insoluble antimicrobial agents, water soluble agents, and enzymes may be present in either the first or second dentifrice compositions. The quaternary ammonium agents, stannous salts, and substituted guanidines are preferably present in the second dentifrice composition. These agents may be present at levels of from about 0.01 % to about 1.5%, by weight of the dentifrice composition.

An herbal agent, including but not limited to, golden thread extract, honeysuckle extract, magnolol, honokiol, and other known herbal agents and mixtures thereof, also may be present in the compositions herein at levels of from about 0.01% to about 0.05%. Such herbal agents are believed to provide antibacterial efficacy. Polyphenols may further be included at levels from about 0.01% to about 2%. A preferred polyphenol is tea polyphenol.

An effective amount of a desensitizing agent may also be incorporated into the present compositions. The desensitizing agents include those selected from alkaline metal salts with a chloride, nitrate sulfate, or acetate of a group II metal or aluminum or polymerizable monomer to occlude the tubules, alkaline metal or ammonium nitrate, ammonium oxylate, citric acid and sodium citrate. Preferred salts are potassium nitrate, potassium citrate, and mixtures thereof. Such desensitizing agents are disclosed in e.g., U.S. Pat. No. 5,718,885.

The dentifrice compositions may be a paste, gel, or any configuration or combination thereof. The dispenser for the dentifrice compositions may be a tube, pump, or any other container suitable for dispensing toothpaste. In a dual phase oral composition, each oral composition will be contained in a physically separated compartment of a dispenser and dispensed side-by-side.

The preferred embodiments of the invention now will be explained in more detail by the following examples. The examples also describe the methodology used in measuring certain properties of the inventive compositions.

### EXAMPLES

### Example 1: Flavor Release Profiling

In order to profile the flavor release, static headspace GC-MS experiments were performed under the following conditions: 2mL of sample was loaded into a 20mL screw cap vial specially designed to prevent leaking of flavor compounds from the headspace air in the vial. The samples were allowed to equilibrate for a set period of time (10min for slurries and 1 hour for toothpastes) in an incubator at 370°C under mild agitation. After equilibration, a 2.5mL headspace syringe was utilized to collect 300µL of headspace air from the flask via automated injection using a Gerstel MPS2 automated injection system. The aliquot of HS air containing volatilized flavor compounds was then injected into an Agilent GC column (Model# 19091S-433) packed with 5% diphenyl-polysiloxane and 95% dimethylpolysiloxane, with column dimensions of 0.25mm × 250µm × 0.25µm. The column was operated in constant flow mode, utilizing an MSD detector and Helium as the carrier gas. The oven temperature was ramped from 40 to 250°C at 20°C/min with an initial hold time at 40°C for 1.5 min. Inlet was set to split-less mode and the total run time was 12.50 min.

Results from conducted static headspace GC-MS experiments show that all samples of the present intention retain anywhere from 8-16% more total flavor than the control slurry, which was a commonly used silica (Zeodent 105). Example 1 with a CTAB of 107 retains the most total flavor compared to the control, roughly 15.80%. Decreasing the CTAB to 89 resulted in a marginal improvement, however, a decrease in CTAB to 79 or lower resulted in a significant improvement in total flavor retention, from 16% to around 8%. See Table 1 below:

**Table 1: Overall flavor sorption/retention by 20 wt% inventive examples (various CTAB values) compared to 20wt% control. All variants retain more flavor than control.**

| Parameter | Example 1 | Example 2 | Example 3* | Example 4* | Control |
|---|---|---|---|---|---|
| Med Particle Size (µm) | 9.3 | 12.6 | 13.1 | 14.0 | 7.0 |
| Oil Absorpt. (cc/200g) | 105.0 | 126.0 | 114.0 | 95.0 | 64.0 |
| CTAB (m²/g) | 107.0 | 89.0 | 79.0 | 65.0 | 30.0 |
| PCR | 94.0 | 93.0 | 91.0 | 92.0 | 113.0 |
| RDA | 102.0 | 76.0 | 80.0 | 89.0 | 211.0 |
| Flavor Conc (ppm) | 124 | 125 | 134 | 131 | 146 |
| Flavor Retention | 15.80% | 14.50% | 8.10% | 8.90% | 6.80% |

| | | | | | |
|---|---|---|---|---|---|
| * outside scope of protection | | | | | |

When total flavor released by the examples is plotted against CTAB in a statistical regression, increasing CTAB surface area strongly correlates with greater retention of flavor compounds (R2= 0.8998). The observed correlation is exponential, explaining why a decrease in CTAB from 107 to 79 leads to a significant improvement, but a further decrease to 60 results in minimal additional improvement. The relatively high accessible internal pore volume of the hybrid silica is believed to allow it to preferentially entrap flavor compounds, which are essentially hydrophobic in nature, resulting in a reduction in the total amount of flavor released.

The hybrid silicas described above are very good at retaining hydrophobic flavor compounds, much better than the control, a commonly used silica (Zeodent 105). This is believed to be due to the smaller pore diameter (∼ 200A) compared to the control d∼593 which leads to a much larger available pore volume for the hydrophobic compounds to interact with. It is also evident from the results presented here that the binding of flavor compounds is preferential, and therefore may be driven by the variations in surface chemistry.

**Table 2: Chemical properties of ten key flavor compounds**

| **Flavor Component** | **Log P_{ow}** | **B.P. (deg C)** | **M.W. (g/mol)** | **Density (g/cm3)** | **Type of Compound** | **Characteristic Aroma** |
|---|---|---|---|---|---|---|
| **Carvone Laevo** | 2.08 | 230-231 | 150.22 | 0.958 | tarpenoid | carraway (-S): spearmint (-R) |
| **Cinn. Aldehyde** | 2.2 | 240 | 132.16 | 1.05 | aromatic aldehyde | spicy cinnamon |
| **Eugenol** | 2.27 | 254 | 164.2 | 1.067 | guaiacol, methoxyphenol | spicy dove |
| **Menthol** | 2.34 | 212 | 156.27 | 0.890 | alcohol | peppermint, cooling |
| **Methyl Salicylate** | 2.51 | 220-224 | 152.15 | 1.174 | salicylic acid methyl ester | wintergreen |
| **Eucalyptol** | 2.74 | 176-177 | 154.25 | 0.9225 | cyclic ether, monoterpene | eucalyptus |
| **Menthone/ iso menthone** | 3.07 | 207 | 154.25 | 0.895 | ketone, monoterpene | peppermint |
| **Anethole** | 3.39 | 234-237 | 148.2 | 0.998 | aromatic unsat. ether | anise |
| **Menthyl acetate** | 3.8 | 228-229 | 198.3 | 0.925 | aromatic ester | fruity-herbaceous minty |
| **Limonene** | 4.35 | 176 | 136.24 | 0.8411 | terpene | citrus, orange |

In order to quantify the flavor release profile as a function of time of the dentifrices, a device was designed to simulate the brushing process and an APCI-MS protocol was developed to measure the intensity of various flavor compounds over time. In this set-up, a water-jacked, completely sealed glass flask serves as the chamber in which the toothpaste and artificial saliva are mixed, as during brushing. The flask has three orifices, one permits air entry into the flask to purge the sample; the second is connected to the APCI-MS for gas analysis and the third is used for the introduction of a brush connected to an electric lab mixer (simulates brushing). This last orifice is firmly closed around the brush shaft with a septum to avoid leaks from the flask. For sampling, a 20 ml plastic syringe is loaded with 15mL of toothpaste gel and introduced into the flask. Thirty milliliters of water are added and the brush is introduced into the flask and connected to the mixer. Once the purge gas flow is then opened (flow rate =100mL/min) the mixer is turned on at 160 rpm. Purging gas facilitates volatile release and a portion enters the inlet of the APCI-MS instrument that is heated to 90 °C to avoid condensation of the compounds.

Once inside the instrument each of the compounds is protonated giving primarily an M⁺¹ ion (M is the molecular ion or parent ion) of a specific intensity. The APCI-MS used for this work allows a second fragmentation of the molecular ion and thus increases the possibility of successful identification and accurate quantification of various compounds, since although two compounds could have the same molecular ion it is extremely unlikely that they will have the same secondary fragmentation pattern. The instrument scans masses between 50 and 200 atomic mass units (uma) every 0.03 seconds and in the time between each full mass scan, the instrument performs fragmentation and scanning of the fragments of specific masses of interest, i.e. corresponding to the molecular ions of the a particular flavor composition. During each experiment data is collected for a total of 3 minutes and each sample is analyzed in triplicates.

At the end of each experiment, the output is collected in the form of aroma release curves (intensity over time) for each flavor compound present. The aroma release curves represent the variation in the intensity of one ion that likely corresponds to one single aroma compound during brushing.

The release of specific flavor compounds varies according to the silica type. The real time release of limonene, menthone, menthol, and carvone from dentifrices made with control as well as with three inventive Examples is shown in **Tables 3-6 below**. The original example (CTAB 107) showed the most pronounced release of all flavor components. Little delay in the release was observed and the maximum intensity was reached in less than 20 seconds in all cases. Example 2 (CTAB 79) showed a pronounced delay in release of menthol (maximum intensity around 30 sec) and a slight delay in the release of menthone and carvone (maximum intensity around 25 sec). The silica's properties can be manipulated as described above to modify flavor release during brushing. Potential applications include flavor morphing and long lasting fresh breath. The data presented in the tables below were estimated from the aroma release curves.

**Table 3: Methone release over time**

| **Menthone Release** | | | | | |
|---|---|---|---|---|---|
| | Release (AU) x 10⁶ time (s) = 0 | Release (AU) x 10⁶ time (s) = 10 | Release (AU) x 10⁶ time (s) = 20 | Release (AU) x 10⁶ time (s) = 30 | Release (AU) x 10⁶ time (s) = 60 |
| Control | 3.30 | 3.20 | 5.60 | 5.20 | 3.90 |
| Example 1 | 3.90 | 3.20 | 9.40 | 6.80 | 3.20 |
| Example 2 | 2.80 | 3.20 | 6.40 | 6.10 | 3.80 |
| Example 4 | 4.00 | 2.40 | 5.20 | 6.50 | 4.40 |

**Table 4: Limonene release over time**

| **Limonene Release** | | | | | |
|---|---|---|---|---|---|
| | Release (AU) x 10⁶ time (s) = 0 | Release (AU) x 10⁶ time (s) = 10 | Release (AU) x 10⁶ time (s) = 20 | Release (AU) x 10⁶ time (s) = 30 | Release (AU) x 10⁶ time (s) = 60 |
| Control | 2.50 | 6.40 | 16.80 | 14.50 | 11.50 |
| Example 1 | 2.50 | 3.80 | 17.20 | 16.00 | 11.00 |
| Example 2 | 2.50 | 4.40 | 18.50 | 16.00 | 11.00 |
| Example 4 | 2.50 | 1.90 | 10.00 | 11.50 | 8.50 |

**Table 5: Menthol release over time**

| **Menthol Release** | | | | | |
|---|---|---|---|---|---|
| | Release (AU) x 106 time (s) = 0 | Release (AU) x 106 time (s) = 10 | Release (AU) x 10⁶ time (s) = 20 | Release (AU) x 106 time (s) = 30 | Release (AU) x 106 time (s) = 60 |
| Control | 4.40 | 4.20 | 6.40 | 6.60 | 5.00 |
| Example 1 | 4.00 | 5.00 | 10.00 | 8.00 | 4.40 |
| Example 2 | 3.60 | 5.00 | 8.40 | 6.80 | 5.00 |
| Example 4 | 5.00 | 3.60 | 7.20 | 9.20 | 7.20 |

**Table 6: Carvone release over time**

| **Carvone Release** | | | | | |
|---|---|---|---|---|---|
| | Release (AU) x 106 time (s) = 0 | Release (AU) x 10⁶ time (s) = 10 | Release (AU) x 106 time (s) = 20 | Release (AU) x 10⁶ time (s) = 30 | Release (AU) x 10⁶ time (s) = 60 |
| Control | 3.30 | 2.30 | 2.70 | 2.50 | 2.05 |
| Example 1 | 3.10 | 2.30 | 5.10 | 3.75 | 2.00 |
| Example 2 | 2.80 | 2.30 | 3.30 | 3.05 | 2.20 |
| Example 4 | 3.30 | 1.40 | 2.05 | 2.65 | 2.00 |

### Example 2: Rheological Testing

The invention further exhibits unexpected viscosity attributes when the hybrid silicas are used in the dentifrice formulation in particular amounts: between 10-30% wt%. This is believed to be due to the unique colloidal network (5 times the G' value relative to a conventional HSC within the linear viscoelastic region at 20% loading) formed by the silicas contained in the dentifrice.

A rheological properties assessment comparing the structural properties and flow behavior of hybrid silicas versus a known high cleaning silica material control supports this result. The high cleaning silica material was Zeodent® 115, commercially available from J.M. Huber Corp., Havre de Grace, Md. The rheology instruments, geometries, and protocols used in this example are well known and documented (*see, e.g.,* [Larson, R.G. (1999) The structure and rheology of complex fluids. *Oxford University Press;* Macosko, C.W. (1994) Rheology: Principles, Measurements, and Applications. Wiley-VCH Inc. ] and references therein).

The linear viscoelastic behavior of various products can be quantified through dynamic oscillatory experiments, i.e. frequency and strain sweeps. In a frequency sweep, the sample was subjected to a small fixed amplitude oscillatory strain (0.05% for all measurements reported here), at different frequencies in the range rads/s. In a strain sweep experiment, the amplitude of the applied strain was varied in the range while the frequency of oscillations was kept constant at 1Hz. The viscoelastic response of the material to this oscillatory strain was measured in terms of G' and G" as a function of frequency or strain, thereby obtaining valuable information regarding the structural properties of the tested materials. The linear viscoelastic region (LVR), for example, can be determined from an oscillatory experiment, as well as the ratio of elastic to viscous contribution (G'/G") often related to the stability and "stiffness" of a particular formulation. With this information, it is possible to determine whether a certain viscoelastic material exhibits more solid-like or more fluid-like properties.

Just as oscillatory shearing can be used to probe the structural and dynamic properties of a sample at equilibrium, steady state shearing also can be used to explore the flow behavior of the system as a function of time, temperature, or shear rate for example. Here, steady state experiments are utilized to quantify the viscosity and shear stress profiles as a function of shear rate in the rage 0.1-100 s-1.

The viscoelastic properties of the invention also are different from those of the control. The low shear viscosity, taken from the viscosity profile at a low shear rate of 0.5s-1 is 161,000cps for the control formula, more than doubles to 361,700cps for the hybrid silica containing formula of the invention. The structural parameter G' (elastic modulus), extracted from the linear viscoelastic region (LVR) of a strain sweep was 11,040 dyn/ cm² and 73,350 dyn/ cm² for the control and Example 1 formulations, respectively, representing an almost one order of magnitude difference. Furthermore, the amount of oscillatory stress required to break up the colloidal network of the control formulation was 29.87 dyn/cm² for the control, when compared to 122.7 dyn/cm² for the hybrid silica formulation of the invention.

The colloidal network formed by the present invention is therefore believed to be stronger and harder to breakdown than the network formed by the control, due to the high-cleaning/ thickening nature of the hybrid silica. These differences in viscosity and structural parameters result in a texture difference perceivable to the consumer and will also affect the rate of flavor release itself.

The latter is evidenced by the flavor release behavior upon dilution. Table 7 below illustrates the total flavor concentration in the headspace as a function of toothpaste dilution with PBS. At 0% dilution, more flavor is released into the headspace from the control formulation but as the sample is diluted by 100 and 200% with PBS the concentration in the headspace becomes higher for the inventive dentifrice formulation. This demonstrates that flavor components that are initially trapped within the strong colloidal network formed by the hybrid silicas are subsequently released upon brushing. The stronger, somewhat delayed release of flavorings results in the stronger burning and numbing sensations post-expectorating that can be achieved with the inventive dentifrice.

**Table 7: Viscosity of dentifrices made from various concentrations of inventive silica**

| | G' (dyn/cm²) @ Silica Conc. = 10 wt% | G' (dyn/cm²) @ Silica Cone. = 20 wt% | G' (dyn/cm²) @ Silica Conc. = 30 wt% |
|---|---|---|---|
| Control | 0 | 0 | 300 |
| Example 1 | 0 | 300 | 7400 |
| Example 2 | 0 | 250 | 5600 |
| Example 4 | 0 | 100 | 1050 |

The unique internal pore structure of the hybrid silica not only has an effect on the total amount of flavor released, it also is believed to affect the rheology profile of the compositions into which the silica materials are incorporated. The effect silica material parameters have on rheology may be important, since the viscosity profile and structural parameters of the final product dictate consumer acceptance and processability of the formulated toothpaste, and impact flavor retention and subsequent release. In aqueous colloidal silica dispersions, the primary particles and clusters are prone to aggregation in the aqueous environment. Accordingly, the size of the existent aggregates may be orders of magnitude higher than that of the dried particles themselves. Above a certain critical volume fraction, the individual clusters interconnect, forming a network of particles that gives the solution its structure and viscosity. The critical particle concentration at which this phenomenon occurs depends, to a large extent, on the pore structure of the material, since it controls the fractal dimension and hence the 'openness' or density of the aggregates.

The rheological properties of three different hybrid silicas are provided in Table 7. Slurries of the hybrid silica were made at three different particle concentration loadings: 10, 20 and 30wt% and compared to control slurries at the same concentrations. Two rheological parameters were measured, the structural parameter G' and a low shear viscosity η₀. An examination of G' and viscosity as a function of silica concentration confirm that all inventive compositions build structure and viscosity faster than the control. At 20% silica concentration, the G' value for the slurry of example 1 is roughly 10 times higher than for the control: 347.6 dyn/cm² versus 40.27 dyn/cm² respectively. Similarly, the low shear viscosity of an inventive dentifrice is 7974cps compared to 1243cps for the control, roughly 7x higher. The more concentrated the slurries are, i.e. 30% by weight silica, these differences become even more pronounced.

Table 7 also indicates that there is a correlation between CTAB surface area and bulk rheological properties. A decrease in CTAB surface area results in a significant improvement in the rheological properties of the invention. While Example 4 with a CTAB value of 65 has a closer rheological profile to the control; the viscosity and structural parameter values are still at least 50% higher. Thus, a one to one replacement of the control silica with the new hybrid silica in the current invention results in a more viscous and more rigid product, conveying a different in-mouth feel to the consumer.

As mentioned above, the pore structure is believed to drive the aggregation behavior of the silica network formed in the aqueous medium, and the formed aggregates are what are believed to ultimately trap flavor, therefore driving flavor retention within the silica network. Results in Table 8 below show this concept. Here, silica slurries were made from four different raw materials ranging in CTAB values. The concentration of all silica slurries was varied from 10-30 weight %, covering a range of concentrations from below the critical aggregation concentration to well above that limit. Results indicate that at 10% silica concentration (below critical aggregation concentration), there is little to no correlation between the total amount of flavor released into the headspace and CTAB surface area (R2 = 0.1625). On the other hand, at 20 and 30% silica concentration there was a strong correlation observed, with a significant decrease in total flavor released resulting from an increase in CTAB surface area.

**Table 8: CTAB values compared with flavor release at varying slurry weight percentages.**

| | CTAB Value | Total Flavor Released (µg/mL) |
|---|---|---|
| 10% wt. slurry | 38 | 152 |
| | 56 | 180 |
| | 79 | 147 |
| | 107 | 180 |
| 20% wt. slurry | 38 | 146 |
| | 56 | 136 |
| | 79 | 135 |
| | 107 | 125 |
| 30% wt. slurry | 38 | 111 |
| | 56 | 96 |
| | 79 | 93.5 |
| | 107 | 80 |

While the silica pore structure is not believed to directly lead to entrapment of flavor; it does appear to affect the extent of aggregation which in turn affects flavor release. This is further reinforced by a strong correlation established between the magnitude of the structural parameter G' and total flavor released. Results indicate that there is a universal exponential correlation between G' and flavor concentration in the headspace, regardless of what silica material is utilized to make the slurry. At low G' values, the flavor release decreases dramatically with increasing G' and eventually plateaus.

While the invention has been described with reference to the detailed description of preferred embodiments, and the preferred examples, a person having ordinary skill in the art will appreciate that the invention is not limited to these preferred embodiments.

## Claims

1. A dentifrice formulation comprising an in situ produced gel/precipitate silica composite, wherein said formulation comprises 10-30% by weight of silica gel/precipitate composite, and the composite exhibits a CTAB surface area of from 85 to 110 m² / g and a ratio of CTAB surface area to oil absorption of from 0.7 to 1.0; and wherein the formulation further comprises an effective amount of flavorant in an amount of up to 0.6% by weight of the formulation.

2. The dentifrice of claim 1 wherein the in situ produced gel/precipitate composite is in the form of particles having a median particle size range between 9 and 14 microns.

3. The dentifrice of claim 1 or claim 2, wherein said composite has a linseed oil absorption of between 80 and 130 ml /100 g.

4. The dentifrice of any one of claims 1 to 3, wherein said composition has a PCR (Pellicle Cleaning Ratio) value of from 81 to 100, a RDA (Radioactive Dentin Abrasion) level of from 75 to 135, and a PCR to RDA ratio of from 0.70 to 1.25.

5. The dentifrice of any one of claims 1 to 4, further comprising an effective amount of a flavor compound wherein the flavor compound exhibits a logarithmic partition (log P) value of 2.0 or greater.

6. The dentifrice of claim 5 wherein the flavor compound is a combination of one or more flavors selected from the group consisting of Carvone Laevo, Cinnamic Aldehyde, Eugenol, Menthol, Methyl Salicylate, Eucalyptol, Menthone, Isomenthone, Anethole, Menthyl Acetate, Limonene, and mixtures thereof.

7. The dentifrice of any one of claims 1 to 6, wherein the dentifrice has a flavor intensity value of about 4.1 or greater.

8. The dentifrice of any one of claims 1 to 7, wherein the dentifrice has a flavor retention value of at least 7.0 %.

9. The dentifrice of any one of claims 1 to 8, wherein the elastic modulus of the dentifrice is within the range of from 65,000 to 100,000 dyn/cm².

10. The dentifrice of any one of claims 1 to 10, further comprising an effective amount of a remineralizing agent.

## Patentansprüche

1. Eine Zahnpflegemittelformulierung, die einen an Ort und Stelle produzierten Gel-/Fällungs-Siliciumdioxid-Verbundstoff beinhaltet, wobei die Formulierung 10-30 Gewichts-% an Siliciumdioxidgel-/Fällungs-Verbundstoff beinhaltet, und der Verbundstoff eine CTAB-Oberfläche von 85 bis 110 m²/g und ein Verhältnis der CTAB-Oberfläche zu der Ölabsorption von 0,7 bis 1,0 zeigt; und wobei die Formulierung ferner eine wirksame Menge an Geschmacksstoff in einer Menge von bis zu 0,6 Gewichts-% der Formulierung beinhaltet.

2. Das Zahnpflegemittel gemäß Anspruch 1, wobei der an Ort und Stelle produzierte Gel-/Fällungs-Verbundstoff in Form von Teilchen mit einem medianen Teilchengrößenbereich zwischen 9 und 14 Mikrometern ist.

3. Das Zahnpflegemittel gemäß Anspruch 1 oder Anspruch 2, wobei der Verbundstoff eine Leinsamenölabsorption von zwischen 80 und 130 ml/100 g aufweist.

4. Das Zahnpflegemittel gemäß einem der Ansprüche 1 bis 3, wobei die Zusammensetzung einen PCR(Pellikel-Reinigungsverhältnis)-Wert von 81 bis 100, ein RDA(Radioaktiver Dentin-Abrieb)-Niveau von 75 bis 135 und ein PCR-zu-RDA-Verhältnis von 0,70 bis 1,25 aufweist.

5. Das Zahnpflegemittel gemäß einem der Ansprüche 1 bis 4, das ferner eine wirksame Menge einer Geschmacksverbindung beinhaltet, wobei die Geschmacksverbindung einen logarithmischen Verteilungs(log-P)-Wert von 2,0 oder mehr zeigt.

6. Das Zahnpflegemittel gemäß Anspruch 5, wobei die Geschmacksverbindung eine Kombination aus einem oder mehreren Geschmäcken ist, ausgewählt aus der Gruppe, bestehend aus Laevo-Carvon, Zimtsäurealdehyd, Eugenol, Menthol, Methylsalicylat, Eucalyptol, Menthon, Isomenthon, Anethol, Menthylacetat, Limonen und Gemischen davon.

7. Das Zahnpflegemittel gemäß einem der Ansprüche 1 bis 6, wobei das Zahnpflegemittel einen Geschmacksintensitätswert von etwa 4,1 oder mehr aufweist.

8. Das Zahnpflegemittel gemäß einem der Ansprüche 1 bis 7, wobei das Zahnpflegemittel einen Geschmacksretentionswert von mindestens 7,0% aufweist.

9. Das Zahnpflegemittel gemäß einem der Ansprüche 1 bis 8, wobei der Elastizitätsmodul der Zahnpflegemittels im Bereich von 65.000 bis 100.000 dyn/ cm² liegt.

10. Das Zahnpflegemittel gemäß einem der Ansprüche 1 bis 10, das ferner eine wirksame Menge eines Remineralisierungsmittels beinhaltet.

## Revendications

1. Formulation de dentifrice comprenant un composite de gel/silice précipitée produit in situ, laquelle dite formulation comprend 10 à 30% en poids d'un composite de gel de silice/silice précipitée, et le composite possède une surface CTAB comprise entre 85 et 110 m² / g et un rapport entre la surface CTAB et l'absorption d'huile compris entre 0,7 et 1,0 ; et laquelle dite formulation comprend en outre une quantité efficace d'essence présente selon une quantité allant jusqu'à 0,6 % en poids de la formulation.

2. Dentifrice selon la revendication 1, le composite gel/ précipité produit in situ se présentant sous forme de particules ayant une dimension granulométrique médiane comprise entre 9 et 14 microns.

3. Dentifrice selon la revendication 1 ou la revendication 2, ledit composite ayant un indice d'absorption de l'huile de lin compris entre 80 et 130 ml/100 g.

4. Dentifrice selon l'une quelconque des revendications 1 à 3, ladite composition ayant une valeur de PCR (coefficient de nettoyage pelliculaire) comprise entre 81 et 100, une valeur de RDA (coefficient d'abrasion de la dentine mesuré par radioactivité) comprise entre 75 et 135, et un rapport PCR sur RDA compris entre 0,70 et 1,25.

5. Dentifrice selon l'une quelconque des revendications 1 à 4, comprenant en outre une quantité efficace d'un composé aromatisant, lequel composé aromatisant présente un coefficient de partage logarithmique (log P) de 2,0 ou supérieur.

6. Dentifrice selon la revendication 5, le composé aromatisant étant une combinaison d'une ou de plusieurs saveurs sélectionnées dans le groupe constitué de carvone Laevo, aldéhyde cinnamique, eugénol, salicylate de méthyle, eucalyptol, menthone, isomenthone, anéthole, acétale de menthyle, limonème et mélanges de ceux-ci.

7. Dentifrice selon l'une quelconque des revendications 1 à 6, le dentifrice ayant un coefficient d'intensité de saveur d'environ 4,1 ou supérieur.

8. Dentifrice selon l'une quelconque des revendications 1 à 7, le dentifrice ayant un coefficient de rétention de saveur d'au moins 7,0 %.

9. Dentifrice selon l'une quelconque des revendications 1 à 8, le module d'élasticité du dentifrice se situant dans la plage comprise entre 65.000 et 100.000 dyn/cm².

10. Dentifrice selon l'une quelconque des revendications 1 à 10, comprenant en outre une quantité efficace d'un agent de reminéralisation.
